# EUROPEAN PATENT APPLICATION

(11) **EP 3 085 359 A1**
(43) Date of publication of application: **26.10.2016**
(21) Application number: 16166700.1
(22) Date of filing: 22.04.2016
(51) Int. Cl.: A61K 9/00, A61K 9/70

(54) **PHOTOSENSITIVE POLYMER FILM BASED ON 5-AMINOLEVULINIC ACID AND ITS DERIVATIVES AND ITS UTILISATION**

(30) Priority: 22.04.2015 CZ 20150272
(71) Applicant: ASKLEPION - Lasercentrum Praha s.r.o., 120 00 Praha 2 (CZ); Rak, Jakub, 323 00 Plzen (CZ); Kral, Vladimir, 120 00 Praha 2 (CZ)
(72) Inventor: Rak, Jakub, 323 00 Plzen (CZ); Král, Vladimír, 120 00 Praha 2 (CZ); Smucler, Roman, 110 00 Praha 1 (CZ)
(74) Representative: Beetz & Partner mbB

(57) **Abstract**

Photosensitive polymer film based on 5-aminolevulinic acid and its derivatives contains one medical layer, at least, and one covering layer, at least, and the medicinal layer contains 2 to 25% weight of 5-aminolevulinic acid and/or its derivative and pharmaceutically acceptable polymer and the covering layer contains pharmaceutically acceptable polymer. These prepared films have specific properties: they are flexible, adhesive to mucosa and to skin, able to fix and to release photosensitizer in a controlled way.

## Description

### Area of the Art

The invention concerns preparation of systems based on polymer carriers for controlled transport of 5-aminolevulinic acid and its derivatives for photodynamic therapy and aesthetic medicine.

### State of the Art

Chewing vegetable drugs is a common habit in many Asian cultures and it results in significant rise of risk of expansion of oral cavity cancer (e.g., oral cavity cancer represents in India almost 40% of all cancer diseases, compared with just 4% in Great Britain). It is 8% of all cancer diseases globally. Oral cavity cancer is treated above all through surgical intervention, other option is irradiation. Most of these tumours are little sensitive to chemotherapy and biological treatment. Surgical treatment usually results in a big drop of life quality because of deformation of important and fine areas of head and neck.

5-aminolevulinic acid (ALA) is an endogenous metabolite that forms usually in mitochondria of succinate-CoA and glycine. Conjugation of eight ALA molecules results of creation of protoporphyrin IX (PpIX) which is subsequently metabolised and finally it results in creation of haem. Conversion of PpIX to its subsequent substrates requires activity of enzyme ferrochelatase and this is the limiting factor concerning rate of ALA metabolise to haem. In case of external administration the conversion ALA to PpIX is quite quick but PpIX cannot be converted with ferrochelatase quickly to the final product haem and thus, PpIX accumulates in cells. Because PpIX is a strong photosensibilizer this metabolic path has been frequently used in the photodynamic therapy (PDT). Because of instability of ALA which is caused by dimerization into pyrazine-2,5-dipropion acid ALA is used in form of relatively hydrophilic hydrochloride. Though PDT based on ALA proved itself to be quite successful in treatment of some types of cancer including skin cancer and many skin diseases, limited diffusion of hydrophilic ALA into a tumour has caused that localisation of ALA in tissue is not optimum. Better bioavailability can be achieved using ALA derivatives, above all in a form of lipophilic esters of ALA which pervade into a lesion better. The most used derivatives are methylester and hexylester of ALA which differ one from another in kinetics of skin penetration and localisation in skin. Other derivatives, salts and salts derivatives have been described but they have not established at the market. The most successful commercial preparations are based on ALA (Aladerm, Porphin), ALA hydrochloride (Gliolan, powder for solution preparation, 1.5 g, Medac; Levulan, 20% solution, DUSA Pharmaceuticals, US Patents: 5,079,262; 5,211,938; 5,954,703; Levulan Kerastics, 20% TOPICAL POWDER FOR SOLUTION Solution; Topical; Aminolevulinic Acid Hydrochloride 23.6%) and ALA methylester hydrochloride (Metvix, cream with 16% mALA.HCl, Galderma; Metvixia, cream with 16.8% mALA.HCl, Galderma).

ALA has established recently as a selected method for superficial basocellular carcinoma which is the most frequent malign tumour. Moreover, its incidence sharply rises with population getting old and with higher sun exposition. Also, interest in prominent aesthetic outcome of this treatment rises, and ALA meets it completely. Thus ALA finds marked potential in aesthetic medicine. PDT using ALA has established in stimulation of replacement of dysplastic cells which together with repair inflammation facilitates marked rejuvenation of skin appearance together with removing efflorescence. Another familiar indication is use in acne treatment. It is useful in monotherapy in light and medium forms, combining with systemic treatment in scar-generating forms.
Commercial preparations for topical applications (solutions, gels and ointments), the therapeutic effect of which and collateral effects depend on experience of the attending physician (quantity of applied ointment, thickness homogeneity of applied layer and the like), require quite experienced personnel and thus even the effect is fortuitous to some extent. Last but not least, we must mention that commercial preparations are not quite suitable for aesthetic medicine because of high concentration of photosensitizer which causes unsubstantiated large damage on skin.
Because of relatively high doses of 5-aminolevulinic acid and its derivatives (5-aminolevulinic acid hydrochloride, 5-aminolevulinic acid esters and 5-aminolevulinic acid esters hydrochlorides), the content of these photosensitizers in commercial preparations uses to be 15-25 weight per cent.

The commercial preparations (solutions, gels and ointments) containing ALA and/or its derivatives can be used for topical application but they are not well applicable for treatment in oral cavity, particularly against oral cavity cancer and also pre-carcinogenic neoplasms where a suitable intervention can prevent development of bodies into tumour diseases. No suitable system for ALA application is available that would keep on wet mucosa for time necessary for diffusion of ALA into tissue to be treated that would not be washed with sputum that would not contain undefined quantity of photosensitizer etc.

### Principle of the Invention

A special photosensitive polymer film containing 2 to 25% of 5-ALA and/or its derivatives has been developed, marked as PSPF(der)ALA, which can be used according to the type of the application and to the reason for treatment for photodynamic treatment/therapy either on the skin for topical application in treatment of skin cancer, treatment of acne and other applications of aesthetic medicine and above all also for mucoadhesive application on wet mucosa for treatment of oral cavity cancer and pre-malignant neoplasms. PSPF(der)ALA contains 5-aminolevulinic acid and/or its derivatives, marked as (der)ALA or their mixture and pharmaceutically acceptable polymer or their mixture. It also can benefit from containing pharmaceutically acceptable plasticizer or their mixture. PSPF(der)ALA meets the most demanding conditions for applications and for persistence on skin and above all on wet mucosa, thus it is adhesive to skin and mucoadhesive to wet mucosa and it is able to release (der)ALA in a defined way into treated tissue where it has been applied. Its composition prevents release of drug out from its therapeutic target. When applied in oral cavity the medicinal layer is protected against sputum and release of active substance into oral cavity. PSPF(der)ALA is a forming compact film of several layers with required mechanic properties. Concentration of 5-ALA and/or its derivatives in PSPF(der)ALA can be selected in range 2% to 25% according to the type of treatment, time of application and irradiation, actual situation and experience of attending physician. The period for treatment can be reduced if increasing the concentration but just taking into account patient's comfort and subsequent photosensitive reaction of the treated area.

PSPF(der)ALA is prepared as multi-layer and each layer always contains pharmaceutically acceptable polymer. The layers are two, at least, and the first layer, at least, is medicinal, thus containing 5-ALA or its derivatives and the last layer is covering. Intermediate layers with varying composition from the medicinal layer to the covering layer can be positioned between the medicinal layer and the covering layer, above all concerning composition of the pharmaceutically acceptable polymer or their mixture and possibly pharmaceutically acceptable plasticizer and they particularly serve to provide good plastic properties.

The derivative 5-aminolevulinic acid is selected in the following group: 5-aminolevulinic acid hydrochloride, 5-aminolevulinic acid ester, 5-aminolevulinic acid ester hydrochloride. The ester of 5-aminolevulinic acid is 5-aminolevulinic acid methylester or 5-aminolevulinic acid hexylester. The ester hydrochloride of 5-aminolevulinic acid is 5-aminolevulinic acid methylester hydrochlorideor 5-aminolevulinic acid hexylester hydrochloride.

The pharmaceutically acceptable polymer is selected in the following group: polyacrylic acid, polyethylenglycol, polypropyleneglycol, block copolymers of polyethylenglycol and of polypropyleneglycol, polysorbate, polyvinylpyrrolidone, polyvinylalcohol, polyacrylamide, poly(isopropylacrylamide), poly(cyclopropylmethacrylamide), ethylcellulose, carboxymethylcellulose, hydroxyethylcellulose, hydroxypropylcellulose, hydroxypropylmethylcellulose, cellulose acetate phtalate, alginic acid, carrageenan, chitosan, hyaluronic acid, pectic acid, copolymers of glycol acid and lactic acid, starch, sodium carboxymethyl starch, polyurethane, silicones, polycarbonate, polychloroprene, polyisobutylene, polycyanoacrylate, polyvinylacetate, t-octylphenoxypolyethoxyethanol, polystyrene, polypropylene, polyvinylchloride, polyethylene, polymethylmethacrylate, polyhydroxyethylmethacrylate, copolymer of acrylic acid and butylacrylate, copolymer of 2-ethylhexyl-acrylate and butylacrylate, copolymer of vinylacetate and methylacrylate, copolymers of ethylenvinylacetate polyethylenterephtalate and polyethylene, copolymer of polyvinylcaprolactam, polyvinylacetate and polyethyleneglycol.

The pharmaceutically acceptable polymer is advantageous if it consists of branched copolymer of polyvinylcaprolactam, polyvinylacetate and polyethylenglycol (Soluplus), of polyethylenglycols with chain of various length (PEG200, PEG6000, PEG40000), polysorbates (Tween 20, Tween 80), polyvinylpyrrolidone (PVP), block copolymers of polyethylenglycol and of polypropyleneglycol (Pluronic F-127, Pluronic F-68).

The pharmaceutically acceptable plasticizer is selected in the following group: acetyltributylcitrate, acetyltriethylcitrate, triethylcitrate, tributylcitrate, trioctylaluminiumcitrate, acetyltrioctylaluminiumcitrate, trihexylcitrate, acetyltuhexylcitrate, butyryltrihexylcitrate, trimethylcitrate, p-terc-butylphenylsalicylate, butylstearate, dibutylsebacate, dietylphtalate, diisobutyladipate, diphenyl-2-ethylhexyl-phosphate, epoxided soybean oil, glycerol monooleate, monoisopropyl citrate, monostearyl citrate, distearyl citrate, tristearyl citrate, triacetin, triethylcitrate, 3-(2-xenolyl)-1,2-epoxypropan.
It is advantageous to select pharmaceutically acceptable plasticizer in the following group: acetyltriethylcitrate, tributylcitrate.

It is advantageous if the covering layer PSPF(der)ALA contains copolymer of polyvinyl-caprolactam, polyvinylacetate and polyethylenglycol.

PSPF(der)ALA have been prepared with incorporated 5-aminolevulinic acid (ALA), 5-aminolevulinic acid hydrochloride (ALA.HCl), 5-aminolevulinic acid methylester (mALA) and 5-aminolevulinic acid methylester hydrochloride (mALA.HCl), containing pharmaceutically acceptable polymer: branched copolymer of polyvinyl-caprolactam, polyvinylacetate and polyethylenglycol (Soluplus), polyethylenglycols with chain of various length (PEG200, PEG6000, PEG40000), polysorbates (Tween 20, Tween 80), polyvinylpyrrolidone (PVP), block copolymers of polyethylenglycol and polypropyleneglycol (Pluronic F-127, Pluronic F-68) and some of them also have contained plasticizer: acethyltriethylencitrate or tributylcitrate.
These prepared PSPF(der)ALA have some slight advantage when compared with other possible mentioned combinations of composition concerning mechanic properties of the film prepared which is more flexible, more compact, not crackling and without need to wet additionally.
The medicinal layer has been prepared in a range of thickness of 0.75 mm to 1,5 mm, with concentration of pure 5-ALA 1.44 to 19.56%.
The intermediate layers have been prepared in a range of thickness of 0.2 mm to 1.25 mm, not containing 5-ALA.
The covering layer has been prepared in a range of thickness of 0.1 mm to 1.8 mm not containing 5-ALA.
The systems based on polymer Soluplus and its mixtures with other pharmaceutically acceptable polymers and pharmaceutically acceptable plasticizers have shown the best properties. Soluplus is a label for branched copolymer of polyvinyl-caprolactam, polyvinylacetate and polyethylenglycol. These prepared films have specific properties: they are flexible, adhesive to mucosa and to skin, and able to fix and release photosensitizer in a controlled way.

PSPF(der)ALA can be used for photodynamic therapy in oral cavity in the form of topical application because its composition provides that it is mucoadhesive, it keeps well on wet mucosa and thus, it facilitates application of drugs in a defined way with controlled release. This enables to quantify duration of effect and to secure that 5-ALA or its derivatives do not permeate into sputum of a patient whose mucosa has been covered with photosensitive polymer film, to reduce duration of applications and to make the intervention more acceptable.
PSPF(der)ALA is prepared in advance, it can be stored.
Experiments have found that photosensitive polymer film keeps in oral application for several hours, it usually keeps longer than it is tolerable for the patient. The usual time of application was 2 hours, with various concentration of 5-ALA and/or their derivatives in the medicinal layer, and the time of application can be changed according to the selected or desired concentration of ALA which permeates slowly into mucosa.

Photosensitive polymer film based on 5-aminolevulinic acid and its derivatives is also suitable for topical applications for photosensitive therapy where again a drug is applied in a defined way with controlled release, without this application markedly depending on the experience of the attending physician and personnel.
Photosensitive polymer film based on 5-aminolevulinic acid and its derivatives can also be used in the field of aesthetic medicine. This application of photosensitizers can be used for treatment of many skin diseases (dermatitis, acne and the like) and skin smoothing with the benefit of controlled release of a drug and use of lower concentration of a drug which does not affect skin that much.
The effect of photosensitive polymer film based on 5-aminolevulinic acid and its derivatives has appeared as subsequent diffusion inflammation-redness, oedema for 3 to 6 days and subsequent unambiguous visible rejuvenating effect on skin. Wrinkles on skin have been reduced, namely concerning their depth and quantity, and the procedure can be repeated after some time gap and the general aesthetic effect can be improved.

The photosensitive polymer film based on 5-aminolevulinic acid and its derivatives is an efficient system for administration of medicaments for local application on skin or administration through mucosa. This film carries the incorporated pharmaceutically active substance, thus 5-aminolevulinic acid and/or its derivative which are released in a controlled way out off the film into skin or mucosa.
Photosensitive polymer film based on 5-aminolevulinic acid and its derivatives has quite good mechanic properties, it is flexible, pliable and adhesive to skin or mucosa. The adhesive properties are very important especially for mucosa. In case of higher content of 5-amino-levulinic acid and/or its derivatives, the properties of polymer film are markedly effected and therefore the composition of the carrier polymer is very important and it cannot be changed arbitrarily without changing mechanic properties.
The task of the covering layer is to prevent release of photosensitizer out off its therapeutic target which is necessary for application in oral cavity. This protection must be satisfied during all the time of film application which uses to be from ten minutes to two hours. Because of different properties and composition of both layers, multilayer polymer carriers can be advantageously utilised when other layers are embedded between the medical layer containing photosensitizer and the protective layer with gradually changing composition passing from composition of the covering layer to composition of the medical layer containing photosensitizer.

The prepared PSPF(der)ALA have been tested concerning mechanic properties (stiffness, flexibility, adhesion), examples of prepared films meeting requirements concerning mechanic properties are presented in examples with description of composition and thickness of the layers.

PSPF(der)ALA with suitable mechanic properties have been tested *in vivo* on mouse and rat models. Adhesion of films containing ALA, ALA.HCl, mALA, mALA.HCl has been studied on animal models. It has been found that the samples keep on skin / in mouth for all the duration of application and they are not dissolved in spit. Adhesion of samples in oral cavity has been studied also with volunteers but films prepared without medication have been used for these experiments.

Transport of the drug into health tissue has been followed indirectly measuring presence and intensity of fluorescence which is shown by drug metabolite - protoporphyrin IX. The fluorescence as depending on time (production of protoporphyrin IX) has been measured as well as its dependence on sample composition and duration of the action. The optimum time of action differed for various samples but generally, it was 5-60 minutes. Also the suitable time for application of irradiation (maximum quantity of protoporphyrin IX in tissue) has differed for various samples and according to duration of application but generally the maximum of fluorescence intensity has appeared in time 1-3 hours from the beginning of administration.

Transport of the drug into tumour tissue has been deduced from necrosis of tumour cells caused by irradiation (photodynamic therapy) which is an indirect proof of accumulation of protoporphyrin IX which is a metabolite of the administered drug. It also has been monitored if tumour recurrence (repeated occurrence of tumour) occurs. Duration of drug application and time of irradiation have been used according to transport into healthy tissue. Complete healing of tumour has occurred in all cases of these samples and no recurrence has been observed within 180 days from treatment application.

The mixtures for preparation of layers have been prepared by diluting their components in suitable solvents and mixing in adequate ratios. The characteristic solvents have been water, methanol, ethanol, possibly dichloromethane, acetone, tetrahydrofurane and chloroform. The solutions have been layered using spraying technique. This procedure has been applied for each layer: first approximately 0.05 mm layer of solution was applied spraying, then solvent was evaporated, then another layer was applied and solvent removed. This procedure was repeated till application of the required carrier layer then the layer thickness was measured. Solvent was removed using three different techniques (nitrogen flow, partial vaccuum, heating) or their combination depending on used solvent or mixture of solvents and properties of solved substances (e.g. temperature stability, volatility).

Thus prepared PSPF(der)ALA is applied on the treated spot at mucosa or skin and allows to adhere. The optimum duration of PSPF(der)ALA action in oral cavity was determined as 120 minutes because reduction of this period caused dramatic drop of treatment effect and on the other hand its extension was beyond the acceptable level of patient's comfort.

We have tested effect of photosensitive polymer film based on 5-aminolevulinic acid and its derivatives namely mucoadhesivity and photodynamic curability pre-malign neoplasms like leucoplakia or erythroplakia (both with high risk for tumour transformation into squamous cell carcinoma) in oral cavity.

After a pre-malign neoplasm had been exposed to the action, complete or partial healing has occurred and the application can be repeated to achieve complete healing of a pre-malign neoplasm. No regress has occurred in case of complete healing and no new development has occurred in case of partial healing within 180 days.
The application was performed under standardized conditions in the PDT area so that the effect could be compared within expert public. Namely the action of photosensitive polymer film based on 5-aminolevulinic acid and its derivatives took 120 minutes then this film was stripped off and the treated spot with diffused 5-ALA and/or its derivative was irradiated for 20 minutes using a source of visible radiation with wavelength 633 nm and output 4 W.

We have also tested effect of photosensitive polymer film based on 5-aminolevulinic acid and its derivatives on superficial basocellular carcinoma on skin. In case of single application of film combined with irradiation complete healing has occurred in most cases and no recurrence, thus repeated appearance of tumour, has been observed within 180 days from treatment application.
We have also tested action of photosensitive polymer film based on 5-aminolevulinic acid and its derivatives in aesthetic medicine where patients have been exposed to effect of photosensitive polymer film based on 5-aminolevulinic acid and its derivatives for skin treatment with the aim to rejuvenate it.
The effect of the preparation has been evident from previous experiments that have shown therapeutic effect which is considered as stronger evidence of effect of the film.
Films were prepared to meet the requirements for mechanical properties. Of all the prepared films solvent was removed in order to ensure long-term stability of the prepared films and especially drug - ALA.
Retention of biologically acceptable solvents, especially water, ethanol, or DMSO, or other dopants allows to achieve suitable mechanical properties, particularly adhesion and flexibility, but at the expense of long-term stability of the prepared films. Adhesion can also be increased by moistening the film before its application e.g. to the skin, preferably for 30 minutes in advance.

### Examples

### Example 1

We have prepared multilayer polymer film with incorporated 5-aminolevulinic acid hydrochloride (ALA.HCl).

For the preparation we have used pharmaceutically acceptable polymers: branched copolymer of polyvinyl-caprolactam, polyvinylacetate and polyethylenglycol (Soluplus). We have prepared the mixtures for preparation of layers through dilution of the components in water and mixing them in adequate weight ratios:
Solution 1:
   solid fraction: 8 weight parts of ALA.HCl : 92 weight parts of SOLUPLUS, it was diluted in water to get 25% solution of solid fraction.
Solution 2:
   solid fraction: SOLUPLUS, it was diluted in water to get 25% solution.

Solution 1 was sprayed stepwise in 0.05 mm layers, then we have always removed the solvent in nitrogen flow and we have repeated the procedure spraying a next batch of solution until the required layer thickness was achieved, namely 1.5 mm. After the last batch of solvent of solution 1 evaporated, solution 2 was applied using the same procedure to depth 1.8 mm. We have controlled thickness of the layers with a micrometer.

The final film had the following composition:

| Layer | Layer Thickness [mm] | Component 1 | Quantity [w. %] | Component 2 | Quantity [w. %] |
|---|---|---|---|---|---|
| 1 | 1.5 | ALA.HCl | 8 | SOLUPLUS | 92 |
| 2 | 1.8 | SOLUPLUS | 100 | | |

### Example 2

We have prepared multilayer polymer film with incorporated 5-aminolevulinic acid methylester (mALA) in oral cavity. For the preparation we have used polymer carriers and plasticizers: branched copolymer of polyvinyl-caprolactam, polyvinylacetate and polyethylenglycol (Soluplus), polyethylenglycol (PEG200), polyvinylpyrrolidone (PVP). We have prepared mixtures for preparation of layers through dilution of the components in water and mixing them in adequate weight ratios.
Solution 1:
   solid fraction: 12 weight parts of ALA.HCl : 75 weight parts of SOLUPLUS, it was diluted in water to get 26% solution of solid fraction.
Solution 2:
   solid fraction: 83 weight parts of SOLUPLUS : 17 weight parts of PEG200, it was diluted in water to get 26% solution.
Solution 3:
   solid fraction: 90 weight parts of SOLUPLUS : 10 weight parts of PEG200, it was diluted in water to get 26% solution.
Solution 4:
   solid fraction: 90 weight parts of SOLUPLUS : 5 weight parts of PEG200 : 5 weight parts of PVP, it was diluted in water to get 26% solution.
Solution 5:
   solid fraction: 90 weight parts of SOLUPLUS : 10 weight parts of PVP, it was diluted in water to get 26% solution.
Solution 6:
   solid fraction: 85 weight parts of SOLUPLUS : 15 weight parts of PVP, it was diluted in water to get 26% solution.
Solution 7:
   solid fraction: 80 weight parts of SOLUPLUS: 20 weight parts of PVP, it was diluted in water to get 26% solution.

We have applied solutions stepwise spraying in 0.05 mm layers, then we have always removed the solvent using light underpressure and we have repeated the procedure spraying a next batch of solution until the required layer thickness of solution was achieved. After the last batch of solvent of solution evaporated we have applied other solution layers using the same procedure always until we have achieved the required thickness. We have controlled thickness of the layers with a micrometer.

The final film had the following composition:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 1.15 | ALA.HCl | 12 | SOLUPLUS | 75 | PEG200 | 13 |
| 2 | 0.2 | SOLUPLUS | 83 | PEG200 | 17 | | |
| 3 | 0.2 | SOLUPLUS | 90 | PEG200 | 10 | | |
| 4 | 0.2 | SOLUPLUS | 90 | PEG200 | 5 | PVP | 5 |
| 5 | 0.2 | SOLUPLUS | 90 | PVP | 10 | | |
| 6 | 0.2 | SOLUPLUS | 85 | PVP | 15 | | |
| 7 | 0.55 | SOLUPLUS | 80 | PVP | 20 | | |

### Example 3

We have prepared multilayer polymer film with incorporated 5-aminolevulinic acid methylester hydrochloride (mALA.HCl) in oral cavity.

For the preparation, we have used pharmaceutically acceptable polymers: branched copolymer of polyvinyl-caprolactam, polyvinylacetate and polyethylenglycol (Soluplus), polyethylenglycols with chain of various length (PEG200, PEG40000) and plasticizer: tributylcitrate. We have prepared mixtures for preparation of layers like in Examples 1 and 2 through dilution of the components in water, so that they would form¹ a 27 % solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.75 | mALA.HCl | 18 | SOLUPLUS | 62 | PEG200 | 20 |
| 2 | 0.12 | SOLUPLUS | 80 | PEG200 | 20 | | |
| 3 | 0.1 | SOLUPLUS | 85 | PEG200 | 15 | | |
| 4 | 0.1 | SOLUPLUS | 90 | PEG200 | 10 | | |
| 5 | 0.1 | SOLUPLUS | 90 | PEG200 | 5 | PEG40000 | 5 |
| 6 | 0.1 | SOLUPLUS | 90 | PEG40000 | 9 | tributylcitrate | 1 |
| 7 | 0.1 | SOLUPLUS | 85 | PEG40000 | 12 | tributylcitrate | 3 |
| 8 | 0.45 | SOLUPLUS | 80 | PEG40000 | 15 | tributylcitrate | 5 |

We have sprayed the solutions in 0.05 mm layers, then we have always removed the solvent through heating it and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 4

We have prepared multilayer polymer film with incorporated 5-aminolevulinic acid methylester hydrochloride (mALA.HCl) in oral cavity.

For the preparation we have used pharmaceutically acceptable polymers: branched copolymer of polyvinyl-caprolactam, polyvinylacetate and polyethylenglycol (Soluplus), polyethyleneglycol (PEG200), block copolymers of polyethylenglycol and polypropyleneglycol (Pluronic F-127, Pluronic F-68), acetyltriethylcitrate. We have prepared mixtures for preparation of layers like in Examples 1 and 2 through dilution of the components in methanol, so that they would form a 20% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.75 | mALA.HCl | 18 | SOLUPLUS | 62 | PEG200 | 20 |
| 2 | 0.12 | SOLUPLUS | 80 | PEG200 | 20 | | |
| 3 | 0.1 | SOLUPLUS | 85 | PEG200 | 15 | | |
| 4 | 0.1 | SOLUPLUS | 90 | PEG200 | 10 | | |
| 5 | 0.1 | SOLUPLUS | 90 | PEG200 | 5 | Pluronic F-127 | 5 |
| 6 | 0.15 | SOLUPLUS | 90 | Pluronic F-127 | 9 | acetyltriethyl -citrate | 1 |
| 7 | 0.15 | SOLUPLUS | 85 | Pluronic F-127 | 12 | acetyltriethyl -citrate | 3 |
| 8 | 0.4 | SOLUPLUS | 80 | Pluronic F-127 | 15 | acetyltriethyl -citrate | 5 |

We have applied solutions for the layers stepwise. We have sprayed the solutions in 0.05 mm layers, then we have always removed the solvent through heating it and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 5

We have prepared multilayer polymer film with incorporated 5-aminolevulinic acid hydrochloride (ALA.HCl) in oral cavity. For the preparation we have used pharmaceutically acceptable polymers: branched copolymer of polyvinyl-caprolactam, polyvinylacetate and polyethylenglycol (Soluplus), polysorbate (Tween 80). We have prepared mixtures for preparation of layers like in Examples 1 and 2 through dilution of the components in water, so that they would form a 24% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.75 | ALA.HCl | 20 | SOLUPLUS | 70 | Tween80 | 10 |
| 2 | 0.1 | SOLUPLUS | 85 | Tween80 | 15 | | |
| 3 | 0.1 | SOLUPLUS | 90 | Tween80 | 10 | | |
| 4 | 0.1 | SOLUPLUS | 95 | Tween80 | 5 | | |
| 5 | 0.35 | SOLUPLUS | 100 | | | | |

We have applied solutions for the layers stepwise. We have sprayed the solutions in 0.05 mm layers then the solvent has always evaporated and we have repeated the procedure spraying the next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 6

We have prepared multilayer polymer film with incorporated 5-aminolevulinic acid methylester (mALA) on skin. For the preparation we have used pharmaceutically acceptable polymers: branched copolymer of polyvinyl-caprolactam, polyvinylacetate and polyethyleneglycol (Soluplus), polysorbate (Tween 20), polyvinylpyrrolidone (PVP). We have prepared mixtures for preparation of layers like in Examples 1 and 2 through dilution of the components in tetrahydrofurane so that they would form a 25% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.8 | mALA | 10 | PVP | 70 | Tween20 | 20 |
| 2 | 0.1 | PVP | 80 | Tween20 | 20 | | |
| 3 | 0.1 | PVP | 85 | Tween20 | 15 | | |
| 4 | 0.1 | PVP | 80 | Tween20 | 15 | SOLUPLUS | 5 |
| 5 | 0.35 | PVP | 80 | Tween20 | 10 | SOLUPLUS | 10 |

We have applied solutions for the layers stepwise. We have sprayed the solutions in 0.05 mm layers then the solvent has always evaporated and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 7

We have prepared multilayer polymer film with incorporated 5-aminolevulinic acid hydrochloride (ALA.HCl) in oral cavity. For the preparation we have used pharmaceutically acceptable polymers: branched copolymer of polyvinyl-caprolactam, polyvinylacetate and polyethylenglycol (Soluplus), polyethylenglycol (PEG200), block copolymers of polyethyleneglycol and polypropyleneglycol (Pluronic F-68). We have prepared mixtures for preparation of layers like in Examples 1 and 2 through dilution of the components in chloro-form so that they would form a 22% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 1.0 | ALA.HCl | 12 | SOLUPLUS | 75 | PEG200 | 13 |
| 2 | 0.25 | SOLUPLUS | 83 | PEG200 | 17 | | |
| 3 | 0.25 | SOLUPLUS | 90 | PEG200 | 10 | | |
| 4 | 0.25 | SOLUPLUS | 90 | PEG200 | 5 | Pluronic F-68 | 5 |
| 5 | 0.25 | SOLUPLUS | 90 | Pluronic F-68 | 10 | | |
| 6 | 0.25 | SOLUPLUS | 85 | Pluronic F-68 | 15 | | |
| 7 | 0.50 | SOLUPLUS | 80 | Pluronic F-68 | 20 | | |

We have applied solutions for the layers stepwise. We have sprayed the solutions in 0.05 mm layers, then the solvent has always evaporated and we have repeated the procedure spraying a next batch of solution until the required layer thickness of the solution has been achieved. After the last batch of solvent of solution evaporated, we have applied other solution layers using the same procedure always until we have achieved the required thickness. We have controlled thickness of the layers with a micrometer.

### Example 8

We have prepared multilayer polymer film with incorporated 5-aminolevulinic acid methylester hydrochloride (mALA.HCl) in oral cavity. For the preparation we have used pharmaceutically acceptable polymers: branched copolymer of polyvinyl-caprolactam, polyvinylacetate and polyethylenglycol (Soluplus), polyethylenglycols (PEG200, PEG6000) and plasticizer: tributylcitrate. We have prepared mixtures for preparation of layers like in Examples 1 and 2 through dilution of the components in water so that they would form a 28% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.75 | mALA.HCl | 18 | SOLUPLUS | 62 | PEG200 | 20 |
| 2 | 0.12 | SOLUPLUS | 80 | PEG200 | 20 | | |
| 3 | 0.1 | SOLUPLUS | 85 | PEG200 | 15 | | |
| 4 | 0.1 | SOLUPLUS | 90 | PEG200 | 10 | | |
| 5 | 0.1 | SOLUPLUS | 90 | PEG200 | 5 | PEG6000 | 5 |
| 6 | 0.1 | SOLUPLUS | 90 | PEG6000 | 9 | tributylcitrate | 1 |
| 7 | 0.1 | SOLUPLUS | 85 | PEG6000 | 12 | tributylcitrate | 3 |
| 8 | 0.6 | SOLUPLUS | 80 | PEG6000 | 15 | tributylcitrate | 5 |

We have applied solutions for the layers stepwise. We have sprayed the solutions in 0.05 mm layers, then the solvent has always evaporated and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 9

We have prepared multilayer polymer film with incorporated 5-aminolevulinic acid (ALA) on skin. For the preparation we have used pharmaceutically acceptable polymers: branched copolymer of polyvinyl-caprolactam, polyvinylacetate and polyethylenglycol (Soluplus), polyethylenglycol (PEG200), block copolymers of polyethylenglycol and polypropyleneglycol (Pluronic F-68). We have prepared mixtures for preparation of layers like in Examples 1 and 2 through dilution of the components in water so that they would form a 19% solution, and we have mixed the components in adequate weight ratios as given in the table for the final composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 1.0 | ALA | 12 | SOLUPLUS | 75 | PEG200 | 13 |
| 2 | 0.25 | SOLUPLUS | 83 | PEG200 | 17 | | |
| 3 | 0.25 | SOLUPLUS | 90 | PEG200 | 10 | | |
| 4 | 0.25 | SOLUPLUS | 90 | PEG200 | 5 | Pluronic F-68 | 5 |
| 5 | 0.25 | SOLUPLUS | 90 | Pluronic F-68 | 10 | | |
| 6 | 0.25 | SOLUPLUS | 85 | Pluronic F-68 | 15 | | |
| 7 | 0.50 | SOLUPLUS | 80 | Pluronic F-68 | 20 | | |

We have sprayed the solutions in 0.05 mm layers then the solvent has always evaporated and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 10

We have prepared multilayer polymer film with incorporated 5-aminolevulinic acid hydrochloride (ALA.HCl) on skin. For the preparation we have used pharmaceutically acceptable polymers: branched copolymer of polyvinyl-caprolactam, polyvinylacetate and polyethylenglycol (Soluplus) and polyvinylpyrrolidone (PVP). We have prepared mixtures for preparation of layers like in Examples 1 and 2 through dilution of the components in water so that they would form a 15% solution, and we have mixed the components in adequate weight ratios as given in the table composition of the film layers:

| Layer | Layer Thickness [mm] | Component 1 | Quantity [w. %] | Component 2 | Quantity [w. %] |
|---|---|---|---|---|---|
| 1 | 1.3 | ALA.HCl | 2 | SOLUPLUS | 98 |
| 2 | 0.2 | SOLUPLUS | 100 | | |
| 3 | 0.2 | SOLUPLUS | 90 | PVP | 10 |

We have applied solutions for the layers stepwise on the substrate. We have applied solutions stepwise spraying in 0.05 mm layers then the solvent has always evaporated under reduced pressure (< 0.2 atm) and slightly increased temperature (< 50°C) and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 11

We have prepared multilayer polymer film with incorporated 5-aminolevulinic acid methylester hydrochloride (mALA.HCl) on skin. For the preparation we have used pharmaceutically acceptable polymers: branched copolymer of polyvinyl-caprolactam, polyvinylacetate and polyethylenglycol (Soluplus) and polyethylenglycol (PEG200). We have prepared mixtures for preparation of layers like in Examples 1 and 2 through dilution of the components in ethanol so that they would form a 21% solution, and we have mixed the components in adequate weight ratios as given in the table composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 1.2 | mALA.HCl | 2 | SOLUPLUS | 80 | PEG200 | 18 |
| 2 | 0.15 | SOLUPLUS | 82 | PEG200 | 18 | | |
| 3 | 0.1 | SOLUPLUS | 85 | PEG200 | 15 | | |
| 4 | 0.1 | SOLUPLUS | 90 | PEG200 | 10 | | |
| 5 | 0.1 | SOLUPLUS | 90 | PEG200 | 5 | | |

We have applied solutions for the layers stepwise on skin. We have applied solutions stepwise spraying in 0.05 mm layers then the solvent has always evaporated under reduced pressure (< 0,5 atm) and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 12

We have prepared multilayer polymer film with incorporated hydrochloridem 5-amino-levulinic acid (ALA.HCl) in oral cavity. For the preparation we have used pharmaceutically acceptable polymers: branched copolymer of polyvinyl-caprolactam, polyvinylacetate and polyethylenglycol (Soluplus), polysorbate (Tween 20), block copolymer of polyethylenglycol and polypropyleneglycol (Pluronic F-127). We have prepared mixtures for preparation of layers like in Examples 1 and 2 through dilution of the components in ethanol so that they would form a 10% solution, and we have mixed the components in adequate weight ratios as given in the table composition of the film layers:

| Layer | Layer Thickness [mm] | Component 1 | Quantity [w. %] | Component 2 | Quantity [w. %] | Component 3 | Quantity [w. %] |
|---|---|---|---|---|---|---|---|
| 1 | 0.7 | ALA.HCl | 25 | SOLUPLUS | 65 | Tween20 | 10 |
| 2 | 0.15 | SOLUPLUS | 87 | Tween20 | 13 | | |
| 3 | 0.1 | SOLUPLUS | 85 | Tween20 | 10 | Pluronic F-127 | 5 |
| 4 | 0.1 | SOLUPLUS | 80 | Tween20 | 10 | Pluronic F-127 | 10 |
| 5 | 0.1 | SOLUPLUS | 80 | Tween20 | 5 | Pluronic F-127 | 15 |
| 6 | 0.15 | SOLUPLUS | 80 | Pluronic F-127 | 20 | | |

We have applied solutions for the layers stepwise on a substrate. We have sprayed the solutions in 0.05 mm layers then the solvent has always evaporated under slightly increased temperature (<50°C) and we have repeated the procedure spraying a next batch of solution until the required layer thickness of the solution has been achieved. After the last batch of solvent of solution evaporated we have applied other solution layers using the same procedure always until we have achieved the required thickness. We have controlled thickness of the layers with a micrometer.

### Example 13

Characterization of the films prepared in Examples 1-9 concerning life on skin and washing in sputum.

We let the applied film as described in the Examples have effect in oral cavity or on skin and then we have stripped it off. We have found that the prepared film has kept well both in oral cavity and on skin during all the period for the planned application and that it almost neither has been washed in sputum nor has it another negative changes. We have attested this fact by measuring the film after application and then after the period of action has lapsed with a micrometer.

| Film | Period of Action [min] | Film Thickness After Application [mm] | Film Thickness After Period of Action [mm] |
|---|---|---|---|
| Example 1 | 40 | 3.3 | 3.0 |
| Example 2 | 60 | 2.7 | 2.5 |
| Example 3 | 30 | 1.82 | 1.72 |
| Example 4 | 30 | 1.87 | 1.76 |
| Example 5 | 80 | 1.4 | 1.2 |
| Example 6 | 100 | 1.45 | 1.21 |
| Example 7 | 110 | 2.75 | 2.5 |
| Example 8 | 50 | 1.97 | 1.77 |
| Example 9 | 40 | 2.75 | 2.75 |

### Example 14

### Summary

Efficiency of the films prepared in Examples above in permeation of (der)ALA into tumour tissue in oral cavity.

Efficiency of the films from above Examples in permeation of into tumour tissue has been determined indirectly from observation of reduction or elimination of tumour tissue after irradiation which is an indirect proof of accumulation of protoporphyrin IX which is a metabolite of the administered drug.

The table below documents ratios between partial and complete healing of tumour tissue in the Examples. The plot shows healing of tumour tissue in % as depending on quantity (der)ALA converted to pure ALA in the medicinal layer.

| Film | Partial Healing (number of cases) | Complete Healing (number of cases) | Number of Tested Subjects | Quantity of Pure ALA in Medicinal Layer (w. %) | Composition of Medicinal Layer (w. %) |
|---|---|---|---|---|---|
| Example 1 + 15 | 8 | 2 | 10 | 6.26 | ALA.HCl (8) SOLUPLUS (92) |
| Example 2 + 16 | 7 | 3 | 10 | 9.39 | ALA.HCl (12) SOLUPLUS (75) PEG200 (13) |
| Example 3 + 17 | 7 | 3 | 10 | 13.00 | mALA.HCl (18) SOLUPLUS (62) PEG200 (20) |
| Example 4 + 18 | 7 | 3 | 10 | 13.00 | mALA.HCl (18) SOLUPLUS (62) PEG200 (20) |
| Example 5 + 19 | 4 | 6 | 10 | 15.65 | ALA.HCl (20) SOLUPLUS (70) Tween80 (10) |
| Example 7 + 20 | 7 | 3 | 10 | 9.39 | ALA.HCl (12) SOLUPLUS (75) PEG200 (13) |
| Example 8 + 21 | 5 | 5 | 10 | 13.00 | mALA.HCl (18) SOLUPLUS (62) PEG200 (20) |
| Example 9 + 22 | 2 | 18 | 20 | 12.00 | ALA (12) SOLUPLUS (75) PEG200 (13) |
| Example 12 + 25 | 2 | 8 | 10 | 19.56 | ALA.HCl (25) SOLUPLUS (65) Tween20 (10) |

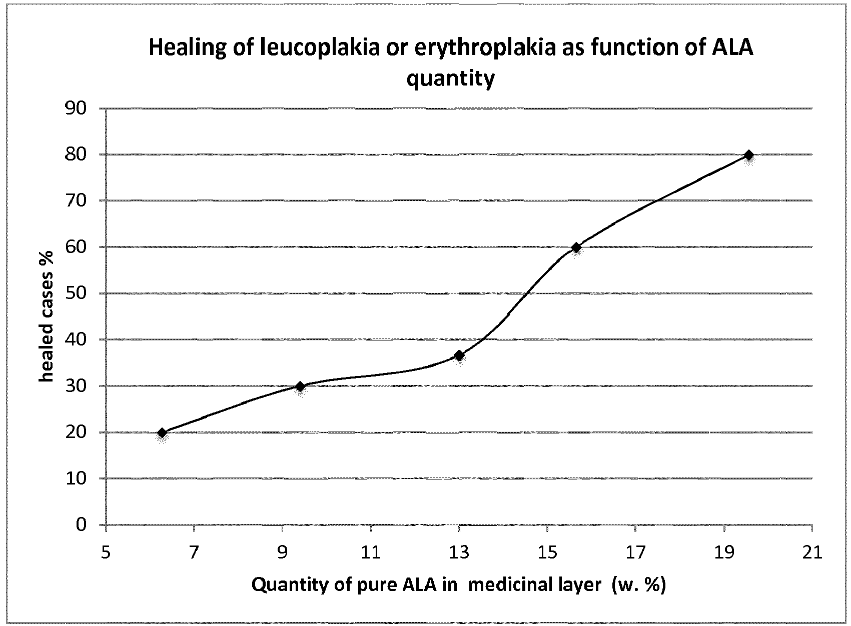

The plot presents average values of healing for the corresponding concentration of pure ALA in medicinal layer.

### Example 15

Application of film prepared in Example 1 - 8% ALA.HCl in Soluplus (6.26% of pure ALA in medicinal layer) combined with irradiation (photodynamic therapy).

The film prepared in Example 1 has been applied on a pre-malign neoplasm of leucoplakia type in ten cases, we have stripped it off after 2 hours incubation and immediately we have irradiated the mucosa with visible light with wavelength 633 nm and output 4W for 20 minutes.

In case of a single use of the film from Example 1 combined with irradiation, partial healing of tumour has occurred in 8 cases and complete healing of tumour has occurred in 2 cases and no recurrence (repeated appearance of tumour) has been observed within 180 days from treatment application.

### Example 16

Application of film prepared in Example 2 - 12% ALA.HCl in Soluplus and PEG200 (9.39% of pure ALA in medicinal layer) combined with irradiation (photodynamic therapy).

The film prepared in Example 2 has been applied on a pre-malign neoplasm of leucoplakia type in ten cases, we have stripped it off after 2 hours incubation and immediately we have irradiated the mucosa with visible light with wavelength 633 nm and output 4W for 20 minutes.

In case of a single use of the film from Example 2 combined with irradiation, partial healing of tumour has occurred in 7 cases and complete healing of tumour has occurred in 3 cases and no recurrence (repeated appearance of tumour) has been observed within 180 days from treatment application.

### Example 17

Application of film prepared in Example 3 - 18% mALA.HCl in Soluplus and PEG200 (13.00% of pure ALA in medicinal layer) combined with irradiation (photodynamic therapy). The film prepared in Example 3 has been applied on a pre-malign neoplasm of the leucoplakia type in ten cases, we have stripped it off after 2 hours incubation and immediately we have irradiated the mucosa with visible light with wavelength 633 nm and output 4W for 20 minutes.

In case of a single use of the film from Example 3 combined with irradiation, partial healing of tumour has occurred in 7 cases and complete healing of tumour has occurred in 3 cases and no recurrence (repeated appearance of tumour) has been observed within 180 days from treatment application.

### Example 18

Application of film prepared in Example 4 - 18% mALA.HCl in Soluplus and PEG200 (13.00% of pure ALA in medicinal layer) combined with irradiation (photodynamic therapy). The film prepared in Example 4 has been applied on a pre-malign neoplasm of the leucoplakia type in ten cases, we have stripped it off after 2 hours incubation and immediately we have irradiated the mucosa with visible light with wavelength 633 nm and output 4W for 20 minutes.

In case of a single use of the film from Example 4 combined with irradiation, partial healing of tumour has occurred in 7 cases and complete healing of tumour has occurred in 3 cases and no recurrence (repeated appearance of tumour) has been observed within 180 days from treatment application.

### Example 19

Application of film prepared in Example 5 - 20% ALA.HCl in Soluplus and Tween80 (15.65% of pure ALA in medicinal layer) combined with irradiation (photodynamic therapy). Film according to Example 5 has been applied on a pre-malign neoplasm of the leucoplakia type in ten cases, we have stripped it off after 2 hours incubation and immediately we have irradiated the mucosa with visible light with wavelength 633 nm and output 4W for 20 minutes.

In case of a single use of the film from Example 5 combined with irradiation, partial healing of tumour has occurred in 4 cases and complete healing of tumour has occurred in 6 cases and no recurrence (repeated appearance of tumour) has been observed within 180 days from treatment application.

### Example 20

Application of film prepared in Example 7 - 12% ALA.HCl in Soluplus and PEG200 (9.39% of pure ALA in medicinal layer) combined with irradiation (photodynamic therapy).

Film according to Example 7 has been applied on a pre-malign neoplasm of the leucoplakia type in ten cases, we have stripped it off after 2 hours incubation and immediately we have irradiated the mucosa with visible light with wavelength 633 nm and output 4W for 20 minutes.

In case of a single use of the film from Example 7 combined with irradiation, partial healing of tumour has occurred in 7 cases and complete healing of tumour has occurred in 3 cases and no recurrence (repeated appearance of tumour) has been observed within 180 days from treatment application.

### Example 21

Application of film prepared in Example 8 - 18% mALA.HCl in Soluplus and PEG200 (13.00% of pure ALA in medicinal layer) combined with irradiation (photodynamic therapy). Film according to Example 8 has been applied on a pre-malign neoplasm of the leucoplakia type in ten cases, we have stripped it off after 2 hours incubation and immediately we have irradiated the mucosa with visible light with wavelength 633 nm and output 4W for 20 minutes.

In case of a single use of the film from Example 8 combined with irradiation, partial healing of tumour has occurred in 5 cases and complete healing of tumour has occurred in 5 cases and no recurrence (repeated appearance of tumour) has been observed within 180 days from treatment application.

### Example 22

Application of film prepared in Example 9 - 12% ALA in Soluplus and PEG200 combined with irradiation (photodynamic therapy).

The film prepared in Example 9 has been applied on superficial basocellular carcinoma on skin in twenty cases, we have stripped it off after 2 hours incubation and immediately we have irradiated the skin with visible light with wavelength 633 nm and output 4W for 20 minutes. In case of a single use of the film according to Example 9 combined with irradiation, partial healing of tumour has occurred in 2 cases and complete healing of tumour has occurred in 18 cases and no recurrence (repeated appearance of tumour) has been observed within 180 days from treatment application.

### Example 23

Application of film (2% of pure ALA in medicinal layer) prepared in Example 10 - 2% ALA.HCl in Soluplus (1.56% of pure ALA in medicinal layer) and film prepared in Example 11 - 2% mALA.HCl in Soluplus and PEG200 (1.44% of pure ALA in medicinal layer) combined with irradiation (aesthetic medicine).

Films prepared in Examples 10 and 11 have been applied on face in twenty cases, we have stripped it off after 60 minutes incubation and immediately we have irradiated the skin with visible light with wavelength 633 nm for 20 minutes.

In case of a single use of the film from Example 10 and 11 combined with irradiation, diffusion inflammation - redness on skin and oedema - has occurred in both cases, it has lasted 3 to 6 days. Then the skin has been assessed. It has looked markedly younger, it has contained less wrinkles and their depth has been lesser. The degree of reduction of wrinkles development has been assessed in both cases after single application 30% in average.

### Example 24

Application of film prepared in Example 12 - 25% mALA.HCl in Soluplus (19.56 % of pure ALA in medicinal layer) combined with irradiation (aesthetic medicine).

Film according to Example 12 has been applied on face in twenty cases, we have stripped it off after 30 minutes incubation and immediately we have irradiated the skin with visible light with wavelength with wavelength 633 nm for 20 minutes.

In case of a single use of the film from Example 12 combined with irradiation diffusion inflammation - redness on skin and oedema - has occurred in both cases, it lasted 3 to 6 days. Then the skin has been assessed. It has looked markedly younger, it has contained less wrinkles and their depth has been lesser. The degree of reduction of wrinkles development has been in both cases after single application assessed 35% in average.

### Example 25

Application of film prepared in Example 12 - 25% mALA.HCl in Soluplus and PEG200 (19.56 % of pure ALA in medicinal layer) combined with irradiation (photodynamic therapy). Film according to Example 12 has been applied on a pre-malign neoplasm of the leucoplakia type in ten cases, we have stripped it off after 2 hours incubation and immediately we have irradiated the mucosa with visible light with wavelength 633 nm and output 4 W for 20 minutes. In case of a single use of the film from Example 12 combined with irradiation, partial healing of tumour has occurred in 2 cases and complete healing of tumour has occurred in 8 cases and no recurrence (repeated appearance of tumour) has been observed within 180 days from treatment application.

### Overview of Therapeutic Effect in Oral Cavity

| Film | Quantity of Pure ALA in Medicinal Layer (w. %) | Composition of Medicinal Layer (w. %) | Period of Film Action Before Irradiation (min) | Duration of Irradiation (min) | Irradiation Wavelength (nm) | Partial /Complete Healing |
|---|---|---|---|---|---|---|
| Example 1+15 | 6.26 | ALA.HCl (8) SOLUPLUS (92) | 120 | 20 | 633 | 8/2 |
| Example 2+16 | 9.39 | ALA.HCl (12) SOLUPLUS (75) PEG200 (13) | 120 | 20 | 633 | 7/3 |
| Example 3+17 | 13.00 | mALA.HCl (18) SOLUPLUS (62) PEG200 (20) | 120 | 20 | 633 | 7/3 |
| Example 4+18 | 13.00 | mALA.HCl (18) SOLUPLUS (62) PEG200 (20) | 120 | 20 | 633 | 7/3 |
| Example 5+19 | 15.65 | ALA.HCl (20) SOLUPLUS (70) Tween80 (10) | 120 | 20 | 633 | 4/6 |
| Example 7+20 | 9.39 | ALA.HCl (12) SOLUPLUS (75) PEG200 (13) | 120 | 20 | 633 | 7/3 |
| Example 8+21 | 13.00 | mALA.HCl (18) SOLUPLUS (62) PEG200 (20) | 120 | 20 | 633 | 5/5 |
| Example 12+25 | 19.56 | ALA.HCl (25) SOLUPLUS (65) Tween20 (10) | 120 | 20 | 633 | 2/8 |

### Overview of Therapeutic Effect on Skin

| Film | Quantity of Pure ALA in Medicinal Layer (w. %) | Composition of Medicinal Layer (w. %) | Period of Film Action Before Irradiation (min) | Duration of Irradiation (min) | Irradiation Wavelength (nm) | Partial /Complete Healing |
|---|---|---|---|---|---|---|
| Example 9+22 | 12.00 | ALA.HCl (12) SOLUPLUS (75) PEG200 (13) | 120 | 20 | 633 | 2/18 |
| Example 10+23 | 1.56 | ALA.HCl (2) SOLUPLUS (98) | 60 | 20 | 633 | Rejuvenation 30% |
| Example 11 + 23 | 1.44 | mALA.HCl (2) SOLUPLUS (80) PEG200 (18) | 60 | 20 | 633 | Rejuvenation 30% |
| Example 12+24 | 19.56 | ALA.HCl (25) SOLUPLUS (65) Tween20 (10) | 30 | 20 | 633 | Rejuvenation 35 % |

### Industrial applicability

Application of photosensitizers based on 5-aminolevulinic acid in oral cavity in the form of mucoadhesive film can be utilised in the pharmaceutical industry for treatment in oral cavity. Topical applications of photosensitizers based on 5-aminolevulinic acid in the form of adhesive film can be utilised in the pharmaceutical industry. Use of films allows applying a drug in a defined way with controlled release. Topical applications of photosensitizers based on 5-aminolevulinic acid in the form of adhesive film can be utilised in the field of aesthetic medicine.

## Claims

1. Photosensitive polymer film based on 5-aminolevulinic acid and its derivatives **characterised by the fact that** it contains one medical layer, at least, and one covering layer, at least, and the medicinal layer contains 2 to 25 % weight of 5-aminolevulinic acid and/or its derivative and pharmaceutically acceptable polymer and the covering layer contains pharmaceutically acceptable polymer, and the pharmaceutically acceptable polymer is selected in the following group: polyacrylic acid, polyethylenglycol, polypropyleneglycol, block copolymers of polyethylenglycol and polypropyleneglycol, polysorbate, polyvinylpyrrolidone, polyvinylalcohol, polyacrylamide, poly(isopropylacrylamide), poly(cyclopropylmethacrylamide), ethylcellulose, carboxymethylcellulose, hydroxyethylcellulose, hydroxypropylcellulose, hydroxypropylmethylcellulose, cellulose acetate phtalate, alginic acid, carrageenan, chitosan, hyaluronic acid, pectic acid, copolymers of glycol acid and lactic acid, starch, sodium carboxymethyl starch, polyurethane, silicones, polycarbonate, polychloroprene, polyisobutylene, polycyanoacrylate, polyvinylacetate, t-octylphenoxypolyethoxyethanol, polystyrene, polypropylene, polyvinylchloride, polyethylene, polymethylmethacrylate, polyhydroxyethylmethacrylate, copolymer of acrylic acid and butylacrylate, copolymer of 2-ethylhexyl-acrylate and butylacrylate, copolymer of vinylacetate and methylacrylate, copolymers of ethylenvinylacetate polyethylenterephtalate and polyethylene, copolymer of polyvinylcaprolactam, polyvinylacetate and polyethylenglycol.

2. Photosensitive polymer film based on 5-aminolevulinic acid and its derivatives according to claim 1 **characterised by the fact that** the derivative 5-aminolevulinic acid is selected in the following group: 5-aminolevulinic acid hydrochloride, 5-aminolevulinic acid ester, 5-aminolevulinic acid ester hydrochloride.

3. Photosensitive polymer film based on 5-aminolevulinic acid and its derivatives according to claim 1 **characterised by the fact that** the pharmaceutically acceptable polymer is selected in the following group: branched copolymer of polyvinylcaprolactam, polyvinylacetate and polyethylenglycol (Soluplus), polyethylenglycols with chain of various length (PEG200, PEG6000, PEG40000), polysorbate (Tween 20, Tween 80), polyvinylpyrrolidone (PVP), block copolymers of polyethylenglycol and polypropyleneglycol (Pluronic F-127, Pluronic F-68)

4. Photosensitive polymer film based on 5-aminolevulinic acid and its derivatives according to claim 2 **characterised by the fact that the** 5-aminolevulinic acid ester is 5-aminolevulinic acid methylester or 5-aminolevulinic acid hexylester.

5. Photosensitive polymer film based on 5-aminolevulinic acid and its derivatives according to claim 2 **characterised by the fact that** the 5-aminolevulinic acid ester hydrochloride is 5-aminolevulinic acid methylester hydrochloride or 5-aminolevulinic acid hexylester hydrochloride.

6. Photosensitive polymer film based on 5-aminolevulinic acid and its derivatives according to claim **1 characterised by the fact that** it also contains one intermediate layer, at least, that is situated between the medical and the covering layers, and the intermediate layer contains pharmaceutically acceptable polymer.

7. Photosensitive polymer film based on 5-aminolevulinic acid and its derivatives according to claim **6 characterised by the fact that** the intermediate layer contains 1 to 5% of pharmaceutically acceptable plasticizer that is selected in the following group: acetyltributylcitrate, acetyltriethylcitrate, triethylcitrate, tributylcitrate, trioctylaluminiumcitrate, acetyltrioctylaluminiumcitrate, trihexylcitrate, acetyltrihexylcitrate, butyryltrihexylcitrate, trimethylcitrate, p-terc-butylphenylsalicylate, butylstearate, dibutylsebacate, dietylphtalate, diisobutyladipate, diphenyl-2-ethylhexyl-phosphate, epoxided soybean oil, glycerol monooleate, monoisopropyl citrate, monostearyl citrate, distearyl citrate, tristearyl citrate, triacetin, triethylcitrate, and 3-(2-xenolyl)-1,2-epoxypropan.

8. Photosensitive polymer film based on 5-aminolevulinic acid and its derivatives according to claim **1 characterised by the fact that** the covering layer contains 1 to 5% of pharmaceutically acceptable plasticizer.

9. Photosensitive polymer film based on 5-aminolevulinic acid and its derivatives according to claim 1 **characterised by the fact that** the pharmaceutically acceptable polymer is copolymer of polyvinylcaprolactam, polyvinylacetate and polyethylenglycol.

10. Photosensitive polymer film based on 5-aminolevulinic acid and its derivatives according to claim **1 characterised by the fact that** the pharmaceutically acceptable polymer is in the form of gel or solution.

11. Photosensitive polymer film based on 5-aminolevulinic acid and its derivatives according to claim 1 **characterised by the fact that** the pharmaceutically acceptable plasticizer is in the form of gel or solution.

12. Photosensitive polymer film based on 5-aminolevulinic acid and its derivatives according to claim 1 for use as a medicament for photosensitive therapy.
